(19) Europäisches Patentamt
European Patent Office
Office européen des brevets

(11) Veröffentlichungsnummer: **0 061 013**
**B1**

(12) # EUROPÄISCHE PATENTSCHRIFT

(45) Veröffentlichungstag der Patentschrift: **27.06.84**

(51) Int. Cl.³: **C 07 C 118/00**, C 07 C 119/042, C 07 C 119/045, C 07 C 119/048

(21) Anmeldenummer: **82101461.0**

(22) Anmeldetag: **26.02.82**

(54) **Verfahren zur Herstellung von Polyisocyanaten.**

(30) Priorität: **10.03.81 DE 3108990**

(43) Veröffentlichungstag der Anmeldung: **29.09.82 Patentblatt 82/39**

(45) Bekanntmachung des Hinweises auf die Patenterteilung: **27.06.84 Patentblatt 84/26**

(84) Benannte Vertragsstaaten: **BE DE FR GB IT NL**

(56) Entgegenhaltungen:
**DE - A - 2 625 075**
**US - A - 3 734 941**

**Chemical Abstracts, Band 91, Nr. 2, 9. Juli 1979, Columbus, Ohio, USA ONODA et al. "Isocyanates" Abstract Nr. 5656g**
**HOUBEN-WEYL "Methoden der organischen Chemie" Sauerstoffverbindungen III, 2. Auflage, 1952, GEORG THIEME VERLAG, Stuttgart Seiten 115 bis 118**

(73) Patentinhaber: **BAYER AG, Konzernverwaltung RP Patentabteilung, D-5090 Leverkusen 1 Bayerwerk (DE)**

(72) Erfinder: **Sundermann, Rudolf, Dr., Treptower Strasse 5, D-5090 Leverkusen (DE)**
Erfinder: **König, Klaus, Dr., Heymannstrasse 50, D-5090 Leverkusen (DE)**
Erfinder: **Engbert, Theodor, Dr., Goethestrasse 57c, D-4047 Dormagen (DE)**
Erfinder: **Becker, Gernot, Dr., Goethestrasse 57, D-4047 Dormagen (DE)**
Erfinder: **Hammen, Günther, Dr., Goethestrasse 67, D-4047 Dormagen (DE)**

**EP 0 061 013 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein verbessertes Verfahren zur Herstellung von Polyisocyanaten durch thermische Spaltung der entsprechenden Carbamate, wobei man die Umsetzung in Gegenwart von Hilfsmitteln, ausgewählt aus der Gruppe bestehend aus Chlorwasserstoff, anorganischen und organischen Säurechloriden, alkylierend wirkenden Verbindungen und Organozinn-IV-chloriden durchführt.

Es ist seit langem bekannt, Isocyanate durch thermische Spaltung von Carbamidsäureestern zu erzeugen (H. Schiff, Ber. der dtsch. chem. Ges., Bd. 3, S. 649 und A. W. Hofmann, Ber. der dtsch. chem. Ges., Bd. 3, S. 653). In der US-PS 2 409 712 ist ein Verfahren beschrieben, bei dem die bei der Spaltung von Carbamidsäureestern entstehenden Produkte durch rasche Destillation oder durch Einleiten in ein Cyclohexan-Wasser-Gemisch an der Rekombination gehindert werden sollen. Das beschriebene Verfahren ist zur diskontinuierlichen Herstellung von Isocyanaten geeignet, führt allerdings nur zu mäßigen Ausbeuten. Außerdem ist es großtechnisch sehr aufwendig.

Ebenfalls in flüssiger Phase, aber in Anwesenheit von inerten Lösungsmitteln verlaufen die Verfahren gemäß den US-PS 3 962 302 und 3 919 278, in denen die thermische Spaltung von mono- und difunktionellen Carbamidsäureestern unter Verzicht auf Zusätze jeglicher Art in einem inerten, hochsiedenden Lösungsmittel erfolgt und die Spaltprodukte Isocyanat und Alkohol kontinuierlich aus dem Reaktionsmedium abdestilliert und separat kondensiert werden. Nachteilig ist bei diesen Verfahren, daß nur mäßige Raum/Zeit-Ausbeuten erreicht werden können.

Zur Erhöhung der Reaktionsgeschwindigkeiten wird in DE-AS 1 016 699, 1 022 222 und 1 028 342 der Einsatz von basischen Katalysatoren vorgeschlagen. Es ist jedoch bekannt (s. z. B. J. Appl. Pol. Sci., Bd. 16, S. 1213), daß basische Katalysatoren zur vermehrten Bildung von festen und unlöslichen Nebenprodukten führen und insofern bei Basenkatalyse nur mäßige Isocyanat-Ausbeuten zu erwarten sind. Darüber hinaus ist die Bildung von festen Nebenprodukten äußerst störend für die technische Durchführung des Spaltprozesses. So können z. B. schwerlösliche Harnstoffe und Isocyanurate zu folgenschweren Verstopfungen von Leitungen und anderen Apparaturteilen führen. Ferner können Kohlendioxid und gasförmige Olefine, die bei der Decarboxylierungsreaktion von Carbamidsäureestern entstehen, starke Gasbelastungen von Destillierkolonnen verursachen.

Ganz besonders wichtig ist die Unterdrückung von Nebenreaktionen bei der Herstellung nicht-destillierbarer Isocyanate, z. B. Polyisocyanat-Gemischen der Diphenylmethanreihe, da in diesem Falle alle nicht-flüchtigen Nebenprodukte im herzustellenden Isocyanat verbleiben und dadurch die Qualität des Isocyanates erheblich verschlechtert werden kann.

In der US-PS 3 919 279 und der DE-OS 2 635 490 sind Verfahren beschrieben, in denen die thermische Spaltung von Carbamidsäureestern in inerten Lösungsmitteln und bei Anwesenheit von Metall-Katalysatoren durchgeführt wird. Die Verwendung dieser zumeist als Lewis-Säuren zu klassifizierenden Verbindungen ist im Vergleich zu basischen Katalysatoren zwar im allgemeinen mit höheren Ausbeuten verbunden; die Bildung von unerwünschten Nebenprodukten ist jedoch nach wie vor nicht ausgeschlossen. Dies bedeutet, daß auch bei Verwendung von nicht-beasischen Katalysatoren mit den erwähnten verfahrenstechnischen und sonstigen Nachteilen gerechnet werden muß.

Mit zusätzlichen schwerwiegenden Nachteilen behaftet sind die in den beiden letztgenannten Patentanschriften beschriebenen katalytischen Verfahren bei der Herstellung von nicht-destillierbarer Isocyanate, sofern die zur Spaltung benutzten Katalysatoren im Spalt-Sumpf verbleiben und sie für die weitere Anwendung des Isocyanats schädlich sind. In der japanischen Patentanmeldung 5 439 002 (1979) wird ein Verfahren zur thermischen Spaltung von TDI-Bisethylurethan vorgestellt, das in einem inerten Lösungsmittel bei Anwesenheit von organischen H-aciden Verbindungen, z. B. $\beta$-Diketonen, aber ohne Metallkatalysatoren, durchgeführt wird. Durch die Zusätze der H-aciden Verbindungen soll die Bildung von unerwünschten Nebenprodukten unterdrückt werden. Bei diesem Verfahren sind wegen des Fehlens wirksamer Katalysatoren allerdings nur relativ geringe Raum/Zeit-Ausbeuten möglich, so daß die Wirtschaftlichkeit dieses Verfahrens insgesamt in Frage zu stellen ist.

Die Aufgabe der vorliegenden Erfindung war es, die Bildung von unerwünschten Nebenprodukten bei der thermischen Spaltung von Urethanen aus mehrfunktionellen Isocyanaten so zu senken, daß ein wirtschaftliches und störungsfrei durchführbares Verfahren zur Herstellung von Isocyanaten erhalten wird.

Diese Aufgabe konnte durch das im folgenden näher erläuterte erfindungsgemäße Verfahren gelöst werden.

Gegenstand der Erfindung ist ein Verfahren zur Herstellung von Polyisocyanaten oder Gemischen von Polyisocyanaten der Formel

$$R^1(NCO)_n$$

durch thermische Spaltung von Carbamaten oder Gemischen von Carbamaten der Formel

$$R^1(NH-CO-O-R^2)_n$$

wobei in diesen Formeln

$R^1$ für einen gegebenenfalls inerte Substituenten aufweisenden und/oder olefinisch ungesättigten aliphatischen Kohlenwasserstoffrest mit insgesamt 2 bis 18 Kohlenstoffatomen, einen gegebenenfalls inerte Substituenten aufweisenden und/oder olefinisch ungesättigten cycloaliphatischen Kohlenwasserstoffrest mit insgesamt 3 bis 18 Kohlenstoffatomen, einem gegebenenfalls inerte Substituenten aufweisenden araliphatischen Kohlenwasserstoffrest mit insgesamt 7 bis 18 Kohlenstoffatomen oder einen gegebenenfalls inerte Substituenten aufweisenden aromatischen Kohlenwasserstoffrest mit insgesamt 6 bis 27 Kohlenstoffatomen steht,

$R^2$ für einen Rest steht, wie er durch Entfernung der Hydroxylgruppe aus einem primären oder sekundären aliphatischen, cycloaliphatischen oder araliphatischen Akohol, dessen Siedepunkt bei Normaldruck mindestens 10°C unter oder über dem Siedepunkt des Polyisocyanats liegt, erhalten wird, wobei im gleichen Molekül des zu spaltenden Carbamats auch unterschiedliche, dieser Definition entsprechende Reste $R^2$ vorliegen können und

n für 2 oder eine ganze Zahl von größer als 2 steht,

gegebenenfalls in Gegenwart von inerten Lösungsmitteln und gegebenenfalls in Gegenwart von Katalysatoren bei Temperaturen von 150 bis 350°C und Drucken von 0,001 bis 20 bar und anschließende Trennung der als Spaltprodukte anfallenden Polyisocyanate und Alkohole, dadurch gekennzeichnet, daß man die thermische Spaltung in Gegenwart von Hilfsmitteln, ausgewählt aus der Gruppe bestehend als Chlorwasserstoff, organischen Säurechloriden, alkylierend wirkenden Substanzen und Organozinn-IV-chloriden durchführt.

Für das erfindungsgemäße Verfahren geeignete Carbamate sind solche der genannten allgemeinen Formel, in welcher $R^1$, $R^2$ und n die genannte Bedeutung haben. Vorzugsweise werden beim erfindungsgemäßen Verfahren solche Carbamate der genannten allgemeinen Formel eingesetzt, für welche

$R^1$ für einen gesättigten aliphatischen Kohlenwasserstoffrest mit 6 bis 10 Kohlenstoffatomen, einen gesättigten cycloaliphatischen Kohlenwasserstoffrest mit 6 bis 15 Kohlenstoffatomen, einen o-, m- oder p-Xylylenrest oder einen gegebenenfalls methylsubstituierten aromatischen Kohlenwasserstoffrest mit insgesamt 6 bis 15 Kohlenstoffatomen steht,

$R^2$ für einen primären oder sekundären aliphatischen Kohlenwasserstoffrest mit 1 bis 4 Kohlenstoffatomen steht;

n für 2 steht.

Im Falle der Verwendung von solchen Carbamaten der genannten allgemeinen Formel, für welche $R^1$ für einen Diphenylen-methyn-Rest steht, wie er formal durch Entfernung der Isocyanatgruppen aus Diisocyanato-diphenylmethan erhalten wird, und für welche n für 2 steht und $R^2$ die genannte Bedeutung hat, können diese Carbamate insbesondere auch im Gemisch mit höheren Homologen eingesetzt werden. Solche Carbamatgemische entstehen beispielsweise bei der säurekatalysierten Kondensation von einfachen Alkoxycarbonylamino-substituierten Benzolen mit Formaldehyd.
Besonders bevorzugt werden beim erfindungsgemäßen Verfahren solche Carbamate der genannten allgemeinen Formel eingesetzt, für welche

$R^1$ für einen Rest steht, wie er durch Entfernung der Isocyanatgruppen aus Hexamethylendiisocyanat, 1-Isocyanato-3,3,5-trimethyl-5-isocyanatomethylcyclohexan (Isophorondiisocyanat), 2,4- und/oder 2,6-Diisocyanatotoluol, 2,4'- und/oder 4,4'-Diisocyanatodiphenylmethan oder Gemischen der letztgenannten beiden Isomeren mit ihren höherkernigen Homologen, d. h. Polyisocyanatgemischen der Diiphenylmethynreihe, erhalten wird und

$R^2$ die zuletzt genannte bevorzugte Bedeutung hat.

Cycloaliphatische Kohlenwasserstoffreste im Sinne dieser Definition sind auch aliphatische Substituenten und/oder Methylenbrücken aufweisende cycloaliphatische Reste wie beispielsweise die dem Isophorondiisocyanat oder die dem 4,4'-Dicyclohexylmethandiisocyanat zugrundeliegenden Kohlenwasserstoffreste. Aromatische Reste im Sinne dieser Definitionen sind auch durch Brückenglieder wie z. B. Methylengruppen, Ethersauerstoffatome oder Sulfongruppen unterbrochene aromatische Reste wie beispielsweise die durch Entfernung der Isocyanatgruppen aus 4,4'-Diisocyanatodiphenylmethan, 4,4'-Diisocyanato-diphenylether oder Diisocyanato-diphenyl-disulfon erhaltenen Reste.

Vorzugsweise werden beim erfindungsgemäßen Verfahren solche Carbamate eingesetzt, deren Alkoholkomponente $R^2$—OH bei Normaldruck einen mindestens 40°C, insbesondere mindestens 70°C unter oder über dem Siedepunkt des Polyisocyanats $R^1(NCO)_n$ liegenden Siedepunkt aufweist. Derartige Siedepunktdifferenzen der Spaltprodukte erleichtern deren destillative Trennung nach der thermischen Spaltung. In der Regel werden beim erfindungsgemäßen Verfahren solche Carbamate eingesetzt, deren Alkoholkomponente bei Normaldruck, wie angegeben, unter dem Siedepunkt des Polyisocyanats $R^1(NCO)_n$ siedet. Die Verwendung von Carbamaten auf Basis von höher als das Polyisocyanat siedenden Alkoholen ist zwar theoretisch möglich, spielt in der Praxis jedoch nur eine untergeordnete Rolle.

Es ist selbstverständlich möglich, beim erfindungsgemäßen Verfahren beliebige Gemische von obigen Definitionen entsprechenden Carbamaten einzusetzen.

Beispiele geeigneter Carbamate sind

1,2-Bis-(cycloheoxycarbonylamino)-ethan, 1,4-Bis-(ethoxycarbonylamino)-butan,
1,6-Bis-(ethoxycarbonylamino)-hexan, 1,8-Bis-(ethoxycarbonylamino)-octan,
1-(Methoxycarbonylamino)-6-(n-butoxycarbonylamino)-hexan,
1-(n-Butoxycarbonylamino)-3,3,5-trimethyl-5-(n-butoxycarbonylamino-methyl)-cyclohexan,
1-(Ethoxycarbonylamino) -3,3,5-trimethyl-5-(ethoxycarbonylamino-methyl)-cyclohexan,
1,4-Bis-(ethoxycarbonylamino)-cyclohexan, 4,4'-Bis-(ethoxycarbonylamino)-dicyclohexylmethan,
4,4'-Bis-(isopropoxycarbonylamino)-dicyclohexylmethan,
1-Methyl-2, 4-bis-(ethoxycarbonylamino)-cyclohexan, 1,3-Bis-(ethoxycarbonylamino)-benzol,
4-bis-(ethoxycarbonylamino)-cyclohexan, 1,3-Bis-(ethoxycarbonylamino)-benzol,
1-Methyl-2,4-bis-(ethoxycarbonylamino)-benzol, 1-Methyl-2,4-(n-butoxycarbonylamino) -benzol,
1-Methyl-2,6-bis-(ethoxycarbonylamino)-benzol, 1,5-Bis-(ethoxycarbonylamino)-naphthalin,
1,3-Bis-(ethoxycarbonylamino)-4-chlor-benzol, 1,4-Bis-(ethoxycarbonylamino-methyl)-benzol,
2,4'-Bis-(ethoxycarbonylamino)-diphenylmethan,
2,4'-Bis-(ethoxycarbonylamino)-diphenylmethan
sowie beliebige Gemische derartiger Carbamate.

Für das erfindungsgemäße Verfahren gut geeignet sind auch Gemische von Carbamaten der Diphenylmethanreihe, beispielsweise Gemische der zuletzt beispielhaft genannten 2,4'- bzw. 4,4'-Bis-(alkoxycarbonylamino)-diphenylmethane mit entsprechenden höherkernigen Homologen, in denen mehr als 2 Alkoxycarbonylamino-substituierte Benzolringe über Methylenbrücken miteinander verknüpft sind. Solche »Carbamatgemische der Diphenylmethanreihe« entstehen beispielsweise bei der säurekatalysierten Kondensation von einfach Alkoxycarbonylamino-substituierten Benzolen mit Formaldehyd.

Die für das erfindungsgemäße Verfahren geeigneten Carbamate sind im übrigen nach bekannten Methoden des Standes der Technik zugänglich. Ihre Herstellung erfolgt beispielsweise durch Umsetzung der entsprechenden Polyamine mit Chlorameisensäureestern, durch Carbonylierung entsprechender Nitroverbindungen in Gegenwart von Alkoholen, durch die bereits erwähnte Kondensationsreaktion von einfachen aromatischen Carbamidsäureestern mit Formaldehyd oder mit anderen Aldehyden oder auch mit Ketonen, oder auch durch Umsetzung von Aminen mit Harnstoff und Alkoholen. Selbstverständlich kommt es bezüglich der Eignung der Carbamate für das erfindungsgemäße Verfahren nicht auf die Art ihrer Herstellung an.

Das erfindungsgemäße Verfahren wird in Gegenwart von erfindungswesentlichen Hilfsmitteln der genannten Art durchgeführt. Diese Hilfsmittel können als »Stabilisatoren« bezeichnet werden, d. h. als Verbindungen, die unerwünschten Neben- und Folgereaktionen entgegenwirken.

Beispiele geeigneter Hilfsmittel der erfindungswesentlichen Art sind

1. Chlorwasserstoff;
2. beliebige organische Säurechloride wie
2.1 Carbonsäurechloride des Molekulargewichtsbereiches 78—500 wie Acetylchlorid, Propionsäurechlorid, Buttersäurechlorid, Pentancarbonsäurechlorid, Valeriansäurechlorid, Laurinsäurechlorid, Ölsäurechlorid, Sterainsäurechlorid, Palmitinsäurechlorid, Chloressigsäurechlorid, Dichloressigsäurechlorid, Chlorpropionsäurechlorid, 2-Methylpropionsäurechlorid, 4-Chlorbuttersäurechlorid, 2-Methylbuttersäurechlorid, 3-Methylbuttersäurechlorid, Dimethylpropionsäurechlorid, 2-Ethylbuttesäurechlorid, 2-Ethylhexansäurechlorid, Methacrylsäurechlorid, Undecensäurechlorid, Linolsäurechlorid, Oxalylchlorid, Bernsteinsäuredichlorid, Glutarsäuredichlorid, Adipinsäuredichlorid, Diethylmalonsäuredichlorid, Octandicarbonsäuredichlorid, Nonandicarbonsäuredichlorid, Trimethyladipinsäuredichlorid, Decandicarbonsäuredichlorid, Dodecandicarbonsäuredichlorid, Heptadecandicarbonsäuredichlorid, Ethoxyessigsäurechlorid, Lävulinsäurechlorid, Cyclohexandicarbonsäuredichlorid, Naphthensäurechlorid, Benzoesäurechlorid, 2-Chlorbenzoesäurechlorid, 3-Chlorbenzoesäurechlorid, 4-Chlorbenzoesäurechlorid, 2,4-Dichlorbenzoesäurechlorid, 2,5-Dichlorbenzoesäurechlorid, 3,4-Dichlorbenzoesäurechlorid, 2-Brombenzoesäurechlorid, 3-Nitrobenzoesäurechlorid, Phenylessigsäurechlorid, 4-Chlorphenylessigsäurechlorid, 2-Methylbenzoesäurechlorid, 3-Methylbenzoesäurechlorid, 4-Methylbenzoesäurechlorid, 4-tert.-Butylbenzoesäurechlorid, 3-Phenyl-2-propensäurechlorid, Abietinsäurechlorid, 1-Naphthalincarbonsäurechlorid, 2-Naphthalincarbonsäurechlorid, Biphenyl-4-carbonsäurechlorid, Camphersäuredichlorid, 5-Norbornen-2,3-dicarbonsäuredichlorid, Perchlor-5-norbornen-2,3-dicarbonsäuredichlorid, Phthalsäuredichlorid, Isophthalsäuredichlorid, Terephthalsäuredichlorid, Tetrachlorphthalsäuredichlorid, 4-Chlorphthalsäuredichlorid, 4-Phenylphthalsäuredichlorid, 1,1'-Binaphthyl-8,8'-dicarbonsäuredichlorid, 1,3,5,-Benzoltricarbonsäuretrichlorid,

1,2,4,5,-Benzoltetracarbonsäuretetrachlorid, 1,4,5,8-Naphthalintetracarbonsäuretetrachlorid, 2-Hydroxybenzoesäurechlorid, 2-Methoxybenzoesäurechlorid, 5-Chlor-2-hydroxybenzoesäurechlorid, Diphenylether-4,4'-dicarbonsäuredichlorid, 3-Chlor-4-hydroxybenzoesäurechlorid, 4-Hydroxyphthalsäuredichlorid;

2.2 Sulfonsäurechlorid des Molekulargewichtsbereichs 114—500, wie Methansulfonsäurechlorid, Chlormethansulfonsäurechlorid, Chlorethansulfonsäurechlorid, Perfluorbutan-1-sulfonsäurechlorid, 4-Chlorbutan-1-sulfonsäurechlorid, Benzolsulfonsäurechlorid, 2-Chlorbenzolsulfonsäurechlorid, 3-Chlorbenzolsulfonsäurechlorid, 4-Chlorbenzolsulfonsäurechlorid, 2,5-Dichlorbenzolsulfonsäuredichlorid, 3,4-Dichlorbenzolsulfonsäuredichlorid, 2-Nitrobenzolsulfonsäurechlorid, 3-Nitrobenzolsulfonsäurechlorid, 4-Nitrobenzolsulfonsäurechlorid, 2-Methylbenzolsulfonsäurechlorid, 3-Methylbenzolsulfonsäurechlorid, 4-Methylbenzolsulfonsäurechlorid, 5-Chlor-2-methylbenzolsulfonsäuredichlorid, 3-Chlor-4-methylbenzolsulfonsäurechlorid, 2,4-Dimethylbenzolsulfonsäurechlorid, Tetralin-6-sulfonsäurechlorid, 1-Naphthalinsulfonsäurechlorid, 2-Naphthalinsulfonsäurechlorid, Biphenyl-4-sulfonsäurechlorid, Pyren-1-fulsonsäurechlorid, Benzol-1,3-disulfonsäuredichlorid, Xylylendisulfonsäuredichlorid, Naphthalin-1,4-disulfonsäuredichlorid, Naphthalin-1,5-disulfonsäuredichlorid, Naphthalin-1,6-disulfonsäuredichlorid, Naphthalin-2,6-disulfonsäuredichlorid, Naphthalin-2,7-disulfonsäuredichlorid, Biphenyl-4,4'-disulfonsäuredichlorid, Naphthalin-1,3,6-trisulfonsäuretrichlorid, Naphthalin-1,3,5,7-tetrasulfonsäuretetrachlorid, Pyren-1,3,6,8-tetrasulfonsäuretetrachlorid, 2-Hoydroxybenzolsulfonsäurechlorid, 4-Hydroxybenzolsulfonsäurechlorid, Diphenylsulfon-3,3'-disulfonsäuredichlorid, Diphenylether-4,4'-disulfonsäuredichlorid, 2,6-Dichlorphenol-4-sulfonsäurechlorid, Phenol-2,4-disulfonsäuredichlorid, Benzoesäurechlorid-2-sulfonsäurechlorid, Benzoesäurechlorid-3-sulfonsäurechlorid, 6-Chlorbenzoesäurechlorid-3-sulfonsäurechlorid, Benzoesäurechlorid-3,5-disulfonsäurechlorid, Phthalsäuredichlorid-4-sulfonsäurechlorid;

2.3 beliebige Carbamidsäurechloride des Molekulargewichtsbereichs 93—500 wie N-Methylcarbamidsäurechlorid, N-Ethylcarbamidsäurechlorid, N-Propylcarbamidsäurechlorid, N-Isopropylcarbamidsäurechlorid, N-(2-Methoxy-ethyl)-carbamidsäurechlorid, N-Butylcarbamidsäurechlorid, N-(2-Phenyl-ethyl)-carbamidsäurechlorid, N-Pentylcarbamidsäurechlorid, N-Neopentylcarbamidsäurechlorid, N-Hexylcarbamidsäurechlorid, N-(6-Chlorhexyl)-carbamidsäurechlorid, N-Octylcarbamidsäurechlorid, N-Heptadecylcarbamidsäurechlorid, N-Allylcarbamidsäurechlorid, N-Cyclohexylcarbamidsäurechlorid, N-(2-Cyclohexyl-cyclohexyl)-carbamidsäurechlorid, N-Benzylcarbamidsäurechlorid, N-Phenylcarbamidsäurechlorid, N-(3,4-Dichlorphenyl)-carbamidsäurechlorid, N-3-Tolylcarbamidsäurechlorid, N-(3-Chlor-4-methyl-phenyl)-carbamidsäurechlorid, N-Cyclohexyl-phenyl)-carbamidsäurechlorid, N-(benzyl-phenyl)-carbarmidsäurechlorid, N-Naphthylcarbamidsäurechlorid, N-Chlornaphthylcarbamidsäurechlorid, 1,4-Butyl-dicarbamidsäurechlorid, 1,6-Hexyl-dicarbamidsäurechlorid, 1, 8-Octyldicarbamidsäurechlorid, Isophoron-dicarbamidsäurechlorid, 1,4-Cyclohexyl-dicarbamidsäurechlorid, 4,4'-Dicyclohexylmethan-dicarbamidsäurechlorid, 2,4-(1-Methylcyclohexyl)-dicarbamidsäurechlorid, 9,10-Anthracen-dicarbamidsäurechlorid, 1,5-Naphthalin-dicarbamidsäurechlorid, 1,3-Benzol-dicarbamisäurechlorid, 1,4-Benzol-dicarbamidsäurechlorid, 2,4-Toluol-dicarbamidsäurechlorid, 2,6-Toluol-dicarbamidsäurechlorid, 1,3-(4-Chlorbenzol)-dicarbamidsäurechlorid, 1,3,5-Benzol-tricarbamidsäurechlorid, Xylylen-dicarbamidsäurechlorid, 4,4'-Diphenylmethan-dicarbamidsäurechlorid;

3. beliebige alkylierend wirkende Substanzen wie

3.1 Sulfonsäurealkylester des Molekulargewichtsbereichs 110—500 wie Methansulfonsäuremethylester, Benzolsulfonsäuremethylester, Toluolsulfonsäuremethylester, ferner u. a. Fluorsulfonsäuremethylester, 1,8-Naphthalinsulfon-6-sulfonsäuremethylester und 4-Ethoxycarbonylbenzolsulfonsäureethylester;

3.2 Dialkylsulfate des Molekulargewichtsbereichs 126—500 wie Dimethylsulfat, Diethylsulfat, Di-(n-Hexyl)-sulfat;

3.3 gegebenenfalls olefinisch ungesättigte Alkylhalogenide des Molekulargewichtsbereichs 50—500 wie Methylchlorid, Methyljodid, Ethyljodid, Ethylbromid, Isopropyljodid, Isopropylbromid, tert.-Butylchlorid, Isobutylchlorid, Isobutylbromid, n-Butylbromid, 1,4-Dichlorbutan, 1-Chlor-3-methylbutan, n-Hexylchlorid, n-Hexylbromid, n-Hexyljodid, n-Heptylchlorid, Dodecylchlorid, Octadecylchlorid, Octadecylbromid, Crotylbromid, Allylbromid, Allylchlorid, 3-Chlor-2-methylpropen, 1,3-Dichlor-2-buten;

3.4 sonstige alkylierend wirkende Ester anorganischer Säuren wie Chlorschwefelsäuremethylester, Cyclohexylbromid, Cyclohexylchlorid, Benzylchlorid, Benzylbromid, 4-Methylbenzylbromid, 4-Methylbenzylchlorid, Xylylenchlorid, Xylylendibromid, 2-Chlor-2-phenylpropan,

6-Chlormethyl-tetralin;

3.5 alkylierend wirkende Ester organischer Säuren wie p-Nitrobenzoesäuremethylester, 3,4-Dinitrobenzoesäuremethylester und Perfluorbotansäuremethylester.

4. Organozinn-IV-chloride wie Dimethyl-zinn-dichlorid, Diethyl-zinn-dichlorid, Dibutyl-zinn-dichlorid, Dihexyl-zinn-dichlorid, Dioctyl-zinn-dichlorid, Dibenzyl-zinn-dichlorid, Dimethyl-chlor-zinn-(2-ethylhexanoat), Dibutyl-chlor-zinn-octanoat, Diethyl-chlor-zinn-acetat, Dimethyl-chlor-zinn-oleat, Dibutyl-chlor-zinnlaurat, Triphenyl-zinn-chlorid, Tributyl-zinn-chlorid, Tribenzyl-zinn-chlorid.

Die erfindungsgswesentlichen Hilfsmittel werden bei der Durchführung des erfindungsgemäßen Verfahrens in Mengen von 0,001 bis 5 Gew.-%, vorzugsweise 0,01 bis 1 Gew.-%, bezogen auf die Gesamtmenge des Reaktionsgemischs incl. der mitverwendeten Lösungsmittel eingesetzt. Die Frage, welchem der beispielshaft genannten Hilfsmittel jeweils der Vorzug gegeben wird ist je nach den Reaktionsbedingungen und der Struktur des zu spaltenden Carbamats zu beantworten. Im allgemeinen ist es günstig, dem Reaktionsgemisch ein solches Hilfsmittel zuzusetzen, das in dem verwendeten Lösungsmittel gut löslich ist und das einen Siedepunkt besitzt, durch den eine ausreichend lange Verweilzeit im Reaktionsgemisch und die leichte Abtrennbarkeit vom herzustellenden Isocyanat gewährleistet sind. Besonders bevorzugt sind solche Hilfsmittel der beispielhaft genannten Art, deren Siedepunkt bei Normaldruck mindestens 50° C unter oder über dem des herzustellenden Polyisocyanats liegt, um eine leichte Abtrennbarkeit des Hilfsmittels vom Verfahrensprodukt zu gewährleisten. Auf diese Einschränkung muß selbstverständlich nicht geachtet werden, falls das eingesetzte Hilfsmittel die weitere Verwendung des Verfahrensproduktes nicht stört, so daß auf seine Abtrennung verzichtet werden kann.

Die Zudosierung des Hilfsmittels zu dem zu spaltenden Carbamat kann nach beliebigen Methoden erfolgen. Vorteilhafterweise erfolgt sie über eine separate Dosiervorrichtung oder auch, unter Verwendung von Lösungen der Hilfsmittel im ohnehin mitverwendeten Lösungsmittel. Bei Verwendung von Chlorwasserstoff oder anderen Hilfsmitteln mit niedrigen Siedepunkten ist es zweckmäßig, derartige Hilfsmittel, gegebenenfalls in Abmischung mit einem Inertgas, das die Funktion eines Trägergases ausüben kann, gasförmig in das Reaktionsgemisch einzuleiten.

Das erfindungsgemäße Verfahren kann insbesondere bei Verwendung von unter den Reaktionsbedingungen flüssigen Carbamaten lösungsmittelfrei durchgeführt werden. Bevorzugt ist jedoch die Mitverwendung geeigneter Lösungsmittel. Als Lösungsmittel kommen beliebige unter den Verfahrensbedingungen flüssige Verbindungen in Betracht, die ein gutes Lösungsvermögen für die zu spaltenden Carbamate besitzen, und die gegenüber den Carbamaten und den erfindungsgemäßen Spaltprodukten inert sind. Besonders gut geeignet sind solche derartige Lösungsmittel, die bei Normaldruck einen Siedepunkt von mindestens 150° C aufweisen. Geeignete Lösungsmittel sind beispielsweise substituierte oder unsubstituierte aromatische Kohlenwasserstoffe wie o-Dichlorbenzol, Dodecylbenzol, Biphenyl, Terphenyl, 4-Chlorbiphenyl, Diphenylether, Biphenyl-phenylether, Phenanthren, Methylnaphthalin, Chlornaphthalin, Dichlornaphthalin, Benzylnaphthalin, Pentoxynaphthalin, Dibenzyltoluol und Dibenzylether; substituierte und unsibstituierte aliphatische Kohlenwasserstoffe wie Dodecan, Octadecan, Hexadecan, 1-Chloroctadecan, Cetylchlorid und Stearylchlorid; Ester organischer und anorganischer Säuren wie Dibutylphthalat, Dioctylphthalat, Benzoesäurebenzylester, Phosphorsäuretriphenylester oder Sulfone wie Diphenylsulfon, Phenyl-tolylsulfon, Naphthyl-phenylsulfon und Tetramethylensulfon.

Die Durchführung des erfindungsgemäßen Verfahrens kann nach verschiedenen Verfahrensvarianten erfolgen, und zwar insbesondere je nachdem, ob das Polyisocyanat als Destillat oder als Sumpfprodukt gewonnen wird.

Bei der Herstellung von schwerflüchtigen Polyisocyanaten wird im allgemeinen das zu spaltende Carbamat zusammen mit den erfindungswesentlichen Hilfsmittel und gegebenenfalls zusammen mit sonstigen Zusätzen als 1- bis 50gew.-%ige, vorzugsweise 1- bis 20gew.-%ige und besonders bevorzugt als 5- bis 10gew.-%ige Lösung in einem oder mehreren Lösungsmitteln der beispielhaft genannten Art in einem geeigneten Reaktionsgefäß bei einem Druck von 0,001 bis 20 bar, vorzugsweise 0,01 bis 1,5 bar, auf 150 bis 350° C, vorzugsweise 180 bis 280° C, erhitzt. Vorzugsweise wird hierbei ein mit einem Dephlegmator versehenes Reaktionsgefäß verwendet und das Lösungsmittel so ausgewählt, daß es unter den genannten Temperatur- und Druckbedingungen siedet. Die hierbei anfallenden Lösungsmitteldämpfe kondensieren im Dephlegmator und fließen in das Reaktionsgefäß zurück. Der entstehende Alkohol passiert gegebenenfalls gleichzeitig mit einem evtl. mitverwendeten Trägergas dampfförmig den Dephlegmator und wird so aus dem Reaktionsgefäß entfernt. Sobald die Spaltung des eingebrachten Carbamats vollständig ist, wird das Lösungsmittel nach bekannten Methoden, z. B. durch Abstreifdestillation, entfernt und das Polyisocyanat als Sumpfprodukt gewonnen. Als Reaktionsgefäß kann bei dieser diskontinuierlichen Arbeitsweise z. B. ein einfacher Kessel verwendet werden.

Bei der erfindungsgemäß bevorzugten Methode zur kontinuierlichen Herstellung von schwerflüchtigen Polyisocanaten wird die genannte Lösung des Carbamats und des Hilfsmittels sowie gegebenenfalls sonstiger Zusatzstoffe innerhalb der genannten Druckbereiche kontinuierlich in einen auf eine

Spalttemperatur innerhalb der genannten Bereiche erhitzten Reaktor geleitet, der entstehende Alkohol, beispielsweise unter Verwendung eines Dephlegmators, in welchem die Lösungsmitteldämpfe kondensieren, gegenbenenfalls unter Zuhilfenahme eines Trägergases, über Kopf abgenommen und die den Spaltreaktor verlassende Produktlösung in geeigneter Weise aufgearbeitet. Die Aufarbeitung erfolgt zweckmäßigerweise ebenfalls kontinuierlich und kann nach jeder bekannten und dafür in Frage kommenden Methoden, z. B. durch Destillation, Dünnschichtung oder Extraktion vorgenommen werden.

Der Spaltreaktor muß derart beschaffen sein, daß die Verweilzeit der eingespeisten Lösung im Reaktor ausreichend lang für eine vollständige Spaltung des Carbamats ist. Kaskadenartig angeordnete Kessel können z. B. diese Bedingung erfüllen.

Die erfindungswesentlichen Hilfsmittel können grundsätzlich in den o. g. Mengen, wie beschrieben, zusammen mit der Lösung des Carbamats in das Reaktionsgefäß eingebracht werden. Es ist jedoch auch möglich, die Hilfsmittel ganz oder teilweise über eigene Dosiervorrichtungen, gegebenenfalls nach Verdünnung mit einem Lösungsmittel, in das Reaktionsgemisch einzudosieren. So kann es zweckmäßig sein, einen Teil des Hilfsmittels erst bei schon fast vollständiger Spaltung des Carbamats oder erst unmittelbar vor dem Aufarbeitungsprozeß zuzugeben.

Sowohl bei der diskontinuierlichen als auch der kontinuierlichen Arbeitsweise erfolgt die erfindungsgemäße thermische Spaltung der Carbamate innerhalb der genannten Druckbereiche, besonders bevorzugt jedoch bei Normaldruck. Die Aufarbeitung der Produktlösung erfolgt hingegen vorzugsweise im Vakuum, beispielsweise bei einem Druck von 1 bis 50 mbar, um die thermische Belastung des Polyisocyanats möglichst niedrig zu halten.

Bei der Herstellung von leicht destillierbaren Polyisocanaten kann analog zu den beschriebenen Methoden der Herstellung von schwerflüchtigen Polyisocyanaten vorgegangen werden, wobei jedoch vorzugsweise das Polyisocyanat nicht bis zur Beendigung des Spaltprozesses im Reaktionsmedium belassen wird, sondern zusammen mit dem Alkohol direkt nach der Spaltung abdestilliert und in der Dampfphase vom Alkohol getrennt wird.

Als besonders gut geeignet zur Herstellung von destillierbaren Polyisocyanaten erwies sich eine Verfahrensweise, nach welcher z. B. das zu spaltende Carbamat als Lösung oder als Schmelze, die gegebenenfalls bereits mit dem erfindungswesentlichen Hilfsmittel vermischt vorliegt, kontinuierlich in einen mit einem geeigneten inerten Lösungsmittel der beispielhaft genannten Art beschickten und auf die Spalttemperatur innerhalb der o. g. Bereiche erhitzten Spaltreaktor eindosiert wird, und die Spaltprodukte, d. h. das Polyisocyanat und der Alkohol durch Einstellen eines entsprechenden Drucks kontinuierlich dem Reaktionsgemisch entzogen und in einer dafür geeigneten Vorrichtung selektiv kondensiert werden. Das hierzu verwendete Reaktionsgefäß kann beispielsweise aus einem einfachen Kessel bestehen, über dem 2 hintereinandergeschaltete und unterschiedlich temperierte Dephlegmatoren angeordnet sind, die beide für das leichter siedende Spaltprodukt durchlässig sind, wobei jedoch der zuoberst angeordnete Dephlegmator auf einer Temperatur gehalten wird, die zwischen den Siedepunkten des Polyisocyanats und des Alkohols liegt. Das am Kopf des unteren Dephlegmators entweichende gasförmige Produktgemisch wird im oberen Dephlegmator so teilkondensiert, daß als Kondensat ein Gemisch aus dem höhersiedenden Spaltprodukt und gegebenenfalls geringen Mengen Lösungsmitteln bzw. geringen Mengen an noch Urethangruppen enthaltendem Material anfällt, und das leichter siedende Spaltprodukt gasförmig am Kopf des oberen Dephlegmators entweicht. Der untere, im allgemeinen unmittelbar auf dem Spaltreaktor angeordnete Dephlegmator soll eine niedrigere Temperatur als das Reaktionsgemisch aufweisen, so daß das gegebenenfalls siedende bzw. das gegebenenfalls vom Produktstrom mitgerissene inerte Lösungsmittel, sowie in die Gasphase gelangende und gegebenenfalls teilgespaltete Carbamate möglichst vollständig an ihm kondensiert werden und in das Reaktionsgefäß zurückfließen.

Bei der erfindungsgemäß bevorzugten Spaltung solcher Carbamate, deren Rest $R^2$ dem Rest eines niedrigsiedenden Alkohols entspricht, wird am oberen Dephlegmator vorwiegend das höher siedende Isocyanat kondensiert und als gasförmiges Kopfprodukt der niedrig siedende Alkohol erhalten, der in einer ausreichend tief gekühlten Vorlage ebenfalls aufgefangen werden kann.

Als für das erfindungsgemäße Verfahren geeignete Dephlegmatoren werden im allgemeinen Wärmeaustauscher verwendet, die mit flüssigen oder gasförmigen Kühlmedien, z. B. Wasser, Wärmeträgeröl oder Luft betrieben werden.

Um die beim Spalzprozeß entstehenden Produkte möglichst rasch und effektiv aus dem Spaltreaktor zu entfernen und um eine Rekombination von Polyisocyanat und Alkohol im Dephlegmatoren-System zu unterdrücken, kann es zweckmäßig sein, durch das Reaktionsgemisch ein inertes Gas oder eine unter Normalbedingungen niedrigsiedende inerte Flüssigkeit, deren Trennung von den Spaltprodukten keine Probleme bereitet, zu leiten. Günstig ist es im allgemeinen auch hier, als Reaktionsmedium ein solches Lösungsmittel einzusetzen, das unter den gewählten Druck- und Temperaturbedingungen gerade siedet.

Das beschriebene Verfahren zur Herstellung destillierbarer Isocyanate kann bei Unter- und Überdruck im Bereich von 0,001 bis 20 bar durchgeführt werden. Bevorzugt wird die Spaltung jedoch bei Unterdruck im Bereich von 0,005 bis 0,5 bar durchgeführt, da dadurch die Gefahr einer Rekombination der Spaltprodukte reduziert wird.

Die Herstellung von reinem Isocyanat aus dem vorwiegend Isocyanat enthaltenden Rohkondensat erfolgt nach bekannten Methoden der Technik, am besten durch fraktionierte Destillation in trennwirksamen Kolonnen. Dabei kann es vorteilhaft sein, die Temperatur im Sumpf der Destillationskolonnen unterhalb der im Spaltreaktor herrschenden zu halten. Dadurch wird eine evtl. thermische Spaltung des im Destillationssumpf vorliegenden Carbamats vermieden, die die Wirksamkeit der Kolonne herabsetzen und die Reinheit des Destillats beeinträchtigen könnte.

Im allgemeinen erfolgt die destillative Abtrennung in der Kolonne bei einer Sumpftemperatur von 40 bis 200°C, vorzugsweise von 80 bis 160°C und einem Druck von 0,001 bis 1,3 bar, bevorzugt 0,005 bis 1,1 bar.

Die bei der destillativen Reindarstellung der Isocyanate $R^1(NCO)_n$ anfallenden Destillationsrückstände bestehen im wesentlichen aus Carbamat und Lösungsmittel sowie gegebenenfalls restlichen Mengen an Polyisocyanat. Sie können in den Spaltreaktor zurückgeführt und dort erneut dem Spaltprozeß unterworfen werden.

Wenn es zweckmäßig erscheint, kann auch aus dem vorwiegend den Alkohol $R^2OH$ enthaltenden Rohkondensat der Alkohol $R^2OH$ abgetrennt und in reiner Form gewonnen werden, und der dabei anfallende Destillationsrückstand, der im wesentlichen wiederum aus Carbamat und Lösungsmittel sowie u. U. restlichen Mengen an Alkohol besteht, in den Spaltreaktor zurückgeführt und erneut dem Spaltprozeß unterworfen werden. Sofern bei der beschriebenen Herstellung der destillerbaren Polyisocyanate in geringem Maße schwerflüchtige Nebenprodukte, die im Reaktionsmedium verbleiben, gebildet werden, können diese auf verschiedene Weise aus dem Reaktionsgemisch abgetrennt werden. Eine Möglichkeit zur Abtrennung solcher Nebenprodukte besteht beispielsweise darin, daß nach einer gewissen Reaktionsdauer, wenn die Konzentration der Nebenprodukte im Reaktionsgemisch zu hoch geworden ist, die Zufuhr von frischem Carbamat unterbrochen, die flüchtigen Anteile des Reaktionsgemisches durch weiteres Abdestillieren möglichst vollständig aus dem Spaltreaktor entfernt und dann das die nicht-flüchtigen Nebenprodukte enthaltende Lösungsmittel abgelassen und durch frisches bzw. wiedergewonnenes, gegebenenfalls schon auf die gewünschte Spalttemperatur vorerhitzte Lösungsmittel, das auch die erfindungsgemäßen Zusätze schon enthalten kann, ersetzt wird.

Wenn es erforderlich oder zweckmäßig erscheint, kann der Rückstand auch kontinuierlich aus dem Reaktionsgemisch ausgeschleust werden. Das läßt sich beispielsweise so bewerkstelligen, daß die Reaktionslösung kontinuierlich aus dem Spaltreaktor entnommen wird und gleichzeitig eine entsprechende Menge an frischem bzw. zurückgewonnenem Lösungsmittel, das schon auf die gewünschte Spalttemperatur erhitzt sein und die erfindungsgemäßen Zusätze enthalten kann, eindosiert wird.

Die Aufarbeitung des ausgeschleusten Lösungsmittels erfolgt nach bekannten Methoden der Technik, z. B. durch Abstreifdestillation. Hierbei kann es vorteilhaft sein, die Destillation bei einer Temperatur vorzunehmen, die oberhalb der im Spaltreaktor herrschenden liegt, da dadurch die Gefahr, daß etwaige restliche Mengen an ebenfalls ausgeschleustem Carbamidsäureester im Destillationssumpf verbleiben, verringert wird. Das destillativ zurückgewonnene Lösungsmittel kann mitsamt der gegebenenfalls mitdestillerten Anteile an Polyisocyanat und/oder Carbamat in den Spaltreaktor zurückgeführt und erneut als Reaktionsmedium verwendet werden.

Wie bereits angedeutet können die erfindungswesentlichen Hilfsmittel dem in das Reaktionsgefäß zuzudosierenden Carbamat beigemischt und/oder zusammen mit dem Lösungsmittel in das Reaktionsgefäß eingebracht und/oder über eine getrennte Dosiervorrichtung in das Reaktionsgemisch eindosiert werden. Die Art und Menge des erfindungswesentlichen Hilfsmittels hängt von der Art des zu spaltenden Carbamats, insbesondere aber von Art und Menge der gegebenenfalls darin vorhandenen Verunreinigungen ab. Sie können durch einfache orientierende Vorversuche ermittelt werden.

Das erfindungsgemäße Verfahren unter Mitverwendung der erfindungswesentlichen Hilfsmittel gestattet die thermische Spaltung von Carbamaten unter Verbesserung der Ausbeute an Polyisocyanaten und unter Verminderung der Menge der als Destillationsrückstand anfallenden, unerwünschten Nebenprodukte.

Das erfindungsgemäße Verfahren kann im übrigen auch unter gleichzeitiger Mitverwendung von weiteren Zusatzstoffen, insbesondere von an sich bekannten Spaltungskatalysatoren, wie sie beispielsweise in der DE-OS 2 635 490 oder der US-PS 3 919 279 beschrieben sind, durchgeführt werden. Diese Katalysatoren werden, falls überhaupt, in Mengen von 0,001 bis 5 Gew.-%, vorzugsweise 0,01 bis 1 Gew.-%, bezogen auf das Gewicht des im Spaltreaktor befindlichen Materials eingesetzt. Vorzugsweise werden solche Katalysatoren des Standes der Technik eingesetzt, deren Siedepunkte deutlich oberhalb der Siedepunkte der Spaltprodukte liegen, die aber noch gegebenenfalls zusammen mit dem als Reaktionsmedium verwendeten inerten Lösungsmittel destilliert und so für einen erneuten Einsatz wiedergewonnen werden können. Im Falle der Herstellung von nicht-destillierbaren Polyisocyanaten empfiehlt sich die Verwendung solcher Katalysatoren, deren vollständige destillative Abtrennung vom im Destillationssumpf verbleibenden Polyisocyanat, gegebenenfalls zusammen mit dem Lösungsmittel, leicht zu bewerkstelligen ist.

Die in den nachfolgenden Beispielen erwähnten Prozentangaben beziehen sich auf Gewichtsprozente.

Die Beispiele 1 bis 9 beschreiben die kontinuierliche Herstellung destillierbarer Polyisocyanate in

Gegenwart der erfindungswesentlichen Hilfsmittel, wobei die mit a) gekennzeichneten Beispiele Vergleichsversuche darstellen, in denen die erfindungsgemäßen Hilfsmittel nicht zur Anwendung kamen. Die Beispiele 10 bis 13 zeigen die erfindungsgemäße Herstellung von schwerflüchtigen Polyisocyanaten, wobei auch hier die mit a) gekennzeichneten Beispiele Vergleichsversuche darstellen.

Beispiel 1

In einen mit 500 g Naphthylphenylsulfon gefüllten 2-Liter-Vierhalskolben, der mit einem effizienten Rührwerk, einer Vorrichtung zum Ablassen des Lösungsmittels, 2 beheizbaren Tropftrichtern und einer aus 2 hintereinandergeschalteten, temperierbaren Dephlegmatoren bestehenden Vorrichtung zur Trennung der gasförmigen Spaltprodukte versehen war, wurde nach Erhitzen des als Reaktionsmedium dienenden Naphthylphenylsulfons auf 250°C kontinuierlich geschmolzenes 2,4-Bis-(ethoxycarbonylamino)-toluol, dem 0,5 Gew.-% Isophthalsäureduchlorid zugemischt war, in einer Rate von etwa 90 g/h eingetropft.

Der Druck in der Spaltapparatur war so eingestellt, daß das 250°C heiße Reaktionsmedium kräftig siedete; er betrug 7,5 mbar.

Das gasförmige Produktgemisch wurde nach Durchgang durch den ersten dephlegmator, der mit Hilfe von Wärmeträgeröl auf 130°C temperiert war und an dem das unter Rückfluß siedende Lösungsmittel kondensierte, am oberen mit Wasser auf 20°C temperierten Dephlegmator so teilkondensiert, daß als Kondensat ein vorwiegend 2,4-Toluylendiisocyanat (TDI) enthaltendes Produkt anfiel. Dieses wurde in einem zwischen den beiden Dephlegmatoren befindlichen Auslaß abgeführt und in einer Vorlage gesammelt.

Das am Kopf des oberen Dephlegmators entweichende Gasgemisch bestand im wesentlichen aus Ethanol und wurde in einer auf —60°C gekühlten Vorlage aufgefangen.

Nach 10 Stunden wurde der Zulauf des Carbamats unterbrochen und als am oberen Dephlegmator kein Produkt mehr kondensierte, das Lösungsmittel abgelassen und durch frisches, schon auf Spalttemperatur vorerhitztes Lösungsmittel ersetzt. Anschließend wurde die Spaltung des Carbamats für weitere 10 Stunden fortgesetzt.

Nachdem insgesamt 1840 g Carbamat auf diese Weise gespalten waren, wurde das vorwiegend aus 2,4-Toluylendiisocyanat bestehende Rohkondensat über eine Füllkörperkolonne destilliert und 940 g (≙78% der Theorie) an reinem 2,4-Toluylendiisocyanat erhalten. Aus der vorwiegend Ethanol enthaltenden Vorlage wurden durch Destillation 516 g (≙81% der Theorie) reines Ethanol erhalten.

Die Destillationssümpfe, die vorwiegend aus Lösungsmittel, unverändertem Ausgangsprodukt (Carbamat) bzw. aus hieraus durch Abspaltung von 1 Mol Ethanol entstandenem Monoisocyanato-monocarbamat bestanden, wurden in der beschriebenen Weise nochmals zur thermischen Spaltung eingesetzt. Aus den dabei erhaltenen Rohkondensaten wurden weitere 150 g 2,4-Toluylendiisocyanat und weitere 86 g Ethanol durch Destillation gewonnen; in den Destillationssümpfen befanden sich noch 52 g unverändertes Ausgangsmaterial (Carbamat) und 46 g Monoisocyanato-monocarbamat.

Zur Rückgewinnung des Lösungsmittels wurde das eingesetzte Naphthylphenylsulfon bei 250°C und 5 mbar ohne Kolonne destilliert; als Rückstand verblieben 105 g eines dunkelbraun gefärbten Feststoffes; das zurückgewonnene Lösungsmittel bestand zu 99,3 Gew.-% aus Naphthylphenylsulfon und zu 0,5 Gew.-% aus Monoisocyanato-monocarbamat.

Die insgesamt nach Beendigung der Reaktion vorhandene, nicht-umgesetzte bzw. durch Rekombination entstandene Menge an Ausgangscarbamat betrug 52 g, die Menge an durch Abspaltung von 1 Mol Alkohol entstandenem Monoisocyanato-monocarbamat lag bei 53 g. Daraus errechnet sich bei Einbeziehung der insgesamt bei der Spaltung entstandenen 1090 g 2,4-Toluylendiisocyanat (≙91% der Theorie) eine Selektivität von 96,7 Mol-% hinsichtlich der Diisocyanatbildung. Demgegenüber läßt sich die Selektivität bezüglich der Ethanolerzeugung zu 100 Mol-% berechnen.

Der insgesamt während der Spaltreaktion entstandene, nicht-destillierbare Rückstand betrug 109 g. Dies entspricht 10 Gew.-%, bezogen auf die Menge an gebildetem Diisocyanat.

Beispiel 1a)

Durchführung wie in Beispiel 1, jedoch ohne Zusatz von Isophthalsäuredichlorid

Ergebnisse:

| | |
|---|---|
| Selektivität bezüglich der TDI-Bildung | 92,1 Mol-% |
| Selektivität bezüglich der Ethanol-Bildung | 100 Mol-% |
| Rückstand, bezogen auf das gebildete TDI | 30 Gew.-% |

Beispiel 2

1500 g 2, 4-Bis-(ethoxycarbonylamino)-toluol wurden wie in Beispiel 1 beschrieben zu TDI und Ethanol gespalten. Als Hilfsmittel wurde Diphenyl-zinn-dichlorid, das dem Lösungsmittel in einer Menge von 0,5 Gew.-%, bezogen auf Lösungsmittel, zugesetzt wurde, eingesetzt. Die Temperatur im Spaltreaktor lag bei 200° C, der Druck bei 5 mbar. Als Lösungsmittel diente Dibenzyltoluol.

Ergebnisse:

| | |
|---|---|
| Selektivität der TDI-Bildung | 98,5 Mol-% |
| Selektivität der Ethanol-Bildung | 99,4 Mol-% |
| Rückstand, bezogen auf das gebildete TDI | 1,5 Gew.-% |

Beispiel 2a)

Durchführung wie in Beispiel 2, jedoch kein Zusatz von Diphenyl-zinn-dichlorid, sondern von 0,5 Gew.-Zinn-II-dioctoat als Katalysator.

Ergebnisse:

| | |
|---|---|
| Selektivität der TDI-Bildung | 86,5 Mol-% |
| Selektivität der Ethanol-Bildung | 100 Mol-% |
| Rückstand, bezogen auf das gebildete TDI | 32 Gew.-% |

Beispiel 3

Wie in Beispiel 1 beschrieben wird 2,4-Bis-(ethoxycabonylamino)-toluol in TDI und Ethanol gespalten. Als Hilfsmittel wurde Isophthalsäuredichlorid verwendet, das man dem Carbamat in einer Menge von 0,5 Gew.-%, bezogen auf Carbamat, zusetzte. Außerdem wurden dem Lösungsmittel 0,5 Gew.-%, bezogen auf Lösungsmittel, Zinn-II-dioctoat zugesetzt. Als Lösungsmittel diente wie im Beispiel 2 Dibenzyltoluol. Die Temperatur im Spaltreaktor betrug wie in Beispiel 2 200° C, der Druck lag ebenfalls bei 5 mbar.

Ergebnisse:

| | |
|---|---|
| Selektivität der TDI-Bildung | 97,2 Mol-% |
| Selektivität der Ethanol-Bildung | 100 Mol-% |
| Rückstand, bezogen auf das gebildete TDI | 12 Gew.-% |

Beispiel 4

Es wird wie in Beispiel 1 beschrieben verfahren. Als Ausgangscarbamat wurde ein Gemisch aus 80% 2,4- und 20% 2,6-Bis-(ethoxycarbonylamino)-toluol verwendet.

Als Erfindungsgemäßes Hilfsmittel wurde Dimethyl-chlor-zinn-(2-ethylhexanoat), das dem Lösungsmittel in einer Menge von 0,1 Gew.-%, bezogen auf Lösungsmittel, zugesetzt wurde, eingesetzt. Als Lösungsmittel diente Dibenzyltoluol. Die Temperatur im Spaltreaktor lag bei 200° C, der Druck bei 5 mbar.

Ergebnisse:

| | |
|---|---|
| Selektivität der TDI-Bildung | 97,3 Mol-% |
| Selektivität der Ethanol-Bildung | 99,2 Mol-% |
| Rückstand, bezogen auf das gebildete TDI | 3,1 Gew.-% |

Beispiel 5

Beispiel 4 wird wiederholt, jedoch unter Verwendung eines Gemisches aus 65% 2,4- und 35% 2,6-Bis-(ethoxycarbonylamino)-toluol und von 0,3 Gew.-%, bezogen auf Lösungsmittel, des in Beispiel 4 genannten Hilfsmittels.

Ergebnisse:

| | |
|---|---|
| Selektivität der TDI-Bildung | 99,7 Mol-% |
| Selektivität der Ethanol-Bildung | 100 Mol-% |
| Rückstand, bezogen auf das gebildete TDI | 1,5 Gew.-% |

Beispiel 6

Beispiel 4 wird wiederholt, jedoch unter Verwendung eines mit 1 Gew.-% Toluylendiamin, bezogen auf Carbamat, verunreinigten Gemischs der in Beispiel 4 genannten Carbamate. Das im Beispiel 4 genannte Hilfsmittel wurde dem Lösungsmittel in einer Menge von 1,5 Gew.-%, bezogen auf Lösungsmittel, einverleibt. Außerdem wurde während des Spaltprozesses kontunierlich eine 10%ige Lösung von Isophthalsäuredichlorid in Dibenzyltouol in das Reaktionsgefäß eingetropft, so daß bezogen auf die Gesamtmenge an eingesetztem Carbamat insgesamt 0,75 Gew.-% Isophthalsäuredichlorid zum Einsatz gelangten.

Ergebnisse:

| | |
|---|---|
| Selektivität der TDI-Bildung | 96,1 Mol-% |
| Selektivität der Ethanol-Bildung | 99,3 Mol-% |
| Rückstand, bezogen auf das gebildete TDI | 10 Gew.-% |

Beispiel 7

Es wird wie in Beispiel 1 verfahren, jedoch als Ausgangscarbamat 1,6-Bis-(ethoxycabonylamino)-hexan eingesetzt. Als Lösungsmittel diente Dibenzyltoluol, dem 1 Gew.-% Diphenyl-zinn-dichlorid zugesetzt wurden. Außerdem wurden dem Carbamat 0,2 Gew.-%, bezogen auf Carbamat, Toluolsulfonsäuremethylester zugesetzt. Die Temperatur im Reaktor lag bei 200° C, der Druck bei 5 mbar.

Ergebnisse:

| | |
|---|---|
| Selektivitäd der Bildung von Hexamethylendiisocyanat (HDI) | 93,6 Mol-% |
| Selektivität der Ethanol-Bildung | 100 Mol-% |
| Rückstand, bezogen auf gebildetes HDI | 23 Gew.-% |

Beispiel 8

Es wird wie in Beispiel 1 beschrieben verfahren, jedoch unter Verwendung von 1-(Ethoxycabonylamino)-3,3,5-trimethyl-5-(ethoxycarbonylamino-methyl)-cyclohexan als Ausgangscarbamat. Dem Carbamat wurden 0,05 Gew.-% Toluolsulfonsäuremethylester zugegeben. Als Lösungsmittel diente Dibenzyltoluol, welchem 1 Gew.-%, bezogen auf Lösungsmittel, Diphenyl-zinn-dichlorid zugesetzt worden war. Die Reaktionstemperatur lag bei 200° C, der Druck bei 5 mbar.

Ergebnisse:

| | |
|---|---|
| Selektivität der Bildung von Isophorondiisocyanat (IPDI) | 98,0 Mol-% |
| Selektivität der Ethanol-Bildung | 100 Mol-% |
| Rückstand, bezogen auf gebildetes IPDI | 3,9 Gew.-% |

Beispiel 9

Beispiel 8 wird wiederholt, jedoch wurde ein mit 1 Gew.-% Isophorondiamin verunreinigtes Ausgangscarbamat eingesetzt. Der Toluolsulfonsäuremethylester wurde nicht dem zu spaltenden Carbamat zugesetzt, sondern separat als 10%ige Lösung in Dibenzyltoluol in den Spaltreaktor eingetropft. Die insgesamt eingetropfte Menge an Toluolsulfonsäuremethylester lag bei 0,6 Gew.-%, bezogen auf Ausgangscarbamat.

Ergebnisse:

| | |
|---|---|
| Selektivität der IPDI-Bildung | 95,8 Mol-% |
| Selektivität der Ethanol-Bildung | 99,5 Mol-% |
| Rückstand, bezogen auf das gebildete IPDI | 12,6 Gew.-% |

Beispiel 10

Eine Lösung von 75 g 4,4'-Bis-(ethoxycarbonylamino)-diphenylmethan in 1425 g Diphenylether wurde unter Zusatz von 0,5 g Terephthalsäuredichlorid bei Normaldruck unter intensivem Rühren auf 250° C erhitzt. Während des Spaltprozesses wurde durch das siedende Reaktionsgemisch getrockneter Stickstoff in einer Rate von 5 bis 10 l/h geleitet und der entstehende Alkohol über eine auf 230° C temperierte Kolonne abdestilliert und in einer gekühlten Vorlage aufgefangen. Die Umsetzung zum Isocyanat wurde durch NCO-Titration von halbstündlich entnommenen Proben bestimmt.

Der maximale NCO-Gehalt der Reaktionslösung war nach 5 Stunden erreicht und betrug 1,23%. Dies entspricht einer Selektivität von 98,9 Mol-%.

Beispiel 10a)

Durchführung wie in Beispiel 10, jedoch kein Terephthalsäuredichlorid eingesetzt.

Ergebnisse:
Selektivität der Bildung von Diisocyanat nach 4 Stunden 91 Mol-%

Beispiel 11

Es wird wie in Beispiel 10 beschrieben verfahren, jedoch ein Gemisch aus Di- und Polycarbamaten der Diphenylmethanreihe mit einem Gehalt von 45 Gew.-% an drei- und höherkernigen Carbamaten eingesetzt, wie es durch Kondensation von Ethoxycarbonylamino-benzol mit Formaldehyd zugänglich ist. Als erfindungswesentliches Hilfsmittel kamen 1,3 Gew.-%, bezogen auf Ausgangscarbamat, an Isophthalsäuredichlorid zum Einsatz.

Ergebnisse:
Selektivität der Polyisocyanatbildung nach 4,5 Stunden 100 Mol-%

Beispiel 12

Beispiel 10 wird wiederholt, jedoch unter Verwendung eines mit 1,3 Gew.-%, bezogen auf Carbamat, 4,4'-Diamino-diphenylmethan verunreinigten Ausgangscarbamats. Als erfindungswesentliches Hilfsmittel wurden 1,33 Gew.-%, bezogen auf Ausgangscarbamat, Isophthalsäuredichlorid mitverwendet.

Ergebnisse:
Selektivität der Bildung des Diisocyanats nach 3 Stunden 96 Mol-%

Beispiel 12a)

Beispiel 12 wird wiederholt, jedoch ohne Mitverwendung des erfindungswesentlichen Zusatzmittels.

Ergebnisse:
Selektivität der Diisocyanatbildung nach 3 Stunden 77 Mol-%

Beispiel 13

In einen als Kesselkaskade konstruierten Spaltreaktor, dessen einzelne durch seitlich angeordnete Überlaufvorrichtungen miteinander verbundene Kessel auf 250°C erhitzt waren, wurde kontinuierlich eine auf 130°C vorerhitzte 5%ige Lösung eines Carbamatsgemischs der Diphenylmethanreihe mit einem Gehalt an höher als 2kernigen Carbamaten von 30 Gew.-%, wie es durch Kondensation von Ethoxycarbonylamino-benzol mit Formaldehyd zugänglich ist, in Diphenylether in einer Menge von 1,0 l/h eingespeist, während gleichzeitig durch die einzelnen Kessel ein Strom getrockneten Stickstoffs in einer Menge von ca. 7,5 l/h durchgeleitet wurde.

Das entstehende Ethanol wurde im Stickstoffstrom aus dem siedenden Reaktionsgemisch herausgetrieben und über auf 230°C temperierte Kolonnen, die direkt oberhalb der Reaktionskessel angeordnet waren, in gekühlte Vorlagen geleitet und dort kondensiert.

Als erfindungswesentliches Hilfsmittel wurde Isophthalsäuredichlorid verwendet, welches in einer Menge von jeweils 1,0 Gew.-%, bezogen auf Carbamat, dem Reaktionsgemisch am Eingang zum ersten und zum dritten Kessel zugesetzt wurde. Nach 7 Stunden befand sich der kontinuierliche Spaltprozeß im Gleichgewicht. Der NCO-Gehalt der den fünften Kessel verlassenden Produktlösung lag zu diesem Zeitpunkt und während anschließender 5 Stunden bei 1,18 Gew.-%. Hieraus errechnet sich eine Selektivität der Polyisocyanatbildung von 99,5 Mol-%.

## Patentansprüche

1. Verfahren zur Herstellung von Polyisocyanaten oder Gemischen von Polyisocyanaten der Formel

$R^1(NCO)_n$

durch thermische Spaltung von Carbamaten oder Gemischen von Carbamaten der Formel

$R^1(NH-CO-O-R^2)_n$

wobei in diesen Formeln

$R^1$ für einen gegebenenfalls inerte Substituenten aufweisenden und/oder olefinisch ungesättigten aliphatischen Kohlenwasserstoffrest mit insgesamt 2 bis 18 Kohlenstoffatomen, einen gegebenenfalls inerte Substituenten aufweisenden und/oder olefinisch ungesättigten cycloaliphatischen Kohlenwasserstoffrest mit insgesamt 3 bis 18 Kohlenstoffatomen, einem gegebenenfalls inerte Substituenten aufweisenden araliphatischen Kohlenwasserstoffrest mit insgesamt 7 bis 18 Kohlenstoffatomen oder einen gegebenenfalls inerte Substituenten aufweisenden aromatischen Kohlenwasserstoffrest mit insgesamt 6 bis 27 Kohlenstoffatomen steht,

$R^2$ für einen Rest steht, wie er durch Entfernung der Hydroxylgruppe aus einem primären oder sekundären aliphatischen, cycloaliphatischen oder araliphatischen Alkohol, dessen Siedepunkt bei Normaldruck mindestens 10°C unter oder über dem Siedepunkt des Polyisocyanats liegt, erhalten wird, wobei im gleichen Molekül des zu spaltenden Carbamats auch unterschiedliche, dieser Definition entsprechende Reste $R^2$ vorliegen können und

n für 2 oder eine ganze Zahl von größer als 2 steht,

gegebenenfalls in Gegenwart von inerten Lösungsmitteln und gegebenenfalls in Gegenwart von Katalysatoren bei Temperaturen von 150 bis 350° C und Drucken von 0,001 bis 20 bar und anschließende Trennung der als Spaltprodukte anfallenden Polyisocyanate und Alkohole, dadurch gekennzeichnet, daß man die thermische Spaltung in Gegenwart von Hilfsmitteln, ausgewählt aus der Gruppe bestehend aus Chlorwasserstoff, organischen Säurechloriden, alkylierend wirkenden Substanzen und Organozinn-IV-chloriden durchführt.

2. Verfahren gemäß Anspruch 1, dadurch gekennzeichnet, daß man als Carbamate solche der in Anspruch 1 genannten Formel einsetzt, für welche

$R^1$ für einen gesättigten aliphatischen Kohlenwasserstoffrest mit 6 bis 10 Kohlenstoffatomen, einen gesättigten cycloaliphatischen Kohlenwasserstoffrest mit 6 bis 15 Kohlenstoffatomen, einen Xylylrest oder einen gegebenenfalls Methyl-Substituenten aufweisenden aromatischen Kohlenwasserstoffrest mit insgesamt 6 bis 15 Kohlenstoffatomen steht,

$R^2$ für einen primären oder sekundären, gesättigten aliphatischen Kohlenwasserstoffrest mit 1 bis 4 Kohlenstoffatomen steht und

n für 2 steht.

3. Verfahren gemäß Anspruch 1, dadurch gekennzeichnet, daß man solche Carbamate der in Anspruch 1 genannten Formel verwendet, für welche

$R^1$ für einen Rest steht, wie er durch Entfernung der Isocyanatgruppen aus Hexamethylendiisocyanat, 1-Isocyanato-3,3,5-trimethyl-5-isocyanatomethyl-cyclohexan, 2,4-Diisocyanatotoluol, 2,6-Diisocyanatotoluol, 2,4'-Diisocyanatodiphenylmethan und/oder 4, 4'-Diisocyanatodiphenylmethan und/oder Gemischen dieser beiden letztgenannten Isomeren mit ihren höheren Homologen erhalten wird,

$R^2$ die in Anspruch 2 genannte Bedeutung hat.

4. Verfahren gemäß Ansprüchen 1 bis 3, dadurch gekennzeichnet, daß man die Hilfsmittel in einer Menge von 0,001 bis 5 Gew.-%, bezogen auf das gesamte Reaktionsgemisch, verwendet.

## Claims

1. A process für the preparation of polyisocyanates or mixtures of polyisocyanates of the formula

$R^1(NCO)_n$

by thermal splitting of carbamates or mixtures of carbamates of the formula

$R^1(NH-CO-O-R^2)_n$

wherein, in these formulae,

$R^1$ represents an aliphatic hydrocarbon radical which optionally contains inert substituents and/or is olefinically unsaturated, with a total of from 2 to 18 carbon atoms, a cycloaliphatic hydrocarbon radical which optionally contains inert substituents and/or is olefinically unsaturated with a total of from 3 to 18 carbon atoms, an araliphatic hydrocarbon radical optionally containing inert substituents with a total of from 7 to 18 carbon atoms or an aromatic hydrocarbon radical optionally containing inert substituents with a total of from 6 to 27 carbon atoms,

$R^2$ represents an radical of the type obtained by removing the hydroxyl group from a primary or secondary aliphatic, cycloaliphatic or araliphatic alcohol of which the boiling point is at least 10°C below or above the boiling point of the polyisocyanate at normal pressure, wherein different radicals $R^2$ corresponding to this definition can also be present in the same molecule of the carbamate to be split; and

n represents 2 or an integer greater than 2,

optionally in the presence of inert solvents and optionally in the presence of catalysts, at temperatures of from 150 to 350°C and pressures of from 0.001 to 20 bar and subsequent separation of the polyisocyanates and alcohols produced as split products, characterised in that the thermal splitting is carried out in the presence of auxiliaries, selected from the group consisting of hydrogen chloride, organic acid chlorides, substances having an alkylation effect and organotin-IV-chlorides.

2. A process according to claim 1, characterised in that carbamates of the formula given in claim 1 are used, in which

$R^1$ represents a saturated aliphatic hydrocarbon radical with from 6 to 10 carbon atoms, a saturated cycloaliphatic hydrocarbon radical with from 6 to 15 carbon atoms, a xylyl radical or an aromatic hydrocarbon radical optionally containing methyl substituents with a total of from 6 to 15 carbon atoms,

$R^2$ represents a primary or secondary saturated aliphatic hydrocarbon radical with from 1 to 4 carbon atoms, and

n represents 2.

3. A process according to claim 1, characterised in that carbamates of the formula given in claim 1 are used, in which

$R^1$ represents a radical of the type obtained by removing the isocyanate groups from hexamethylene diisocyanate, 1-isocyanato-3,3,5-trimethyl-5-isocyanatomethyl-cyclohexane, 2,4-diisocyanato-toluene, 2,6-diisocyanato-toluene, 2,4'-diisocyanatodiphenylmethane and/or 4,4'-diisocyanatodiphenylmethane and/or mixtures of these last two mentioned isomers with their higher homologues, and

$R^2$ has the meaning given in claim 2.

4. A process according to claims 1 to 3, characterised in that the auxiliaries are used in a quantity of from 0.001 to 5% by weight, based on the total reaction mixture.

**Revendications**

1. Procédé de production de polyisocyanates ou de mélange de polyisocyanates de formule

$$R^1(NCO)_n$$

par thermolyse de carbamates ou de mélange de carbamates de formule

$$R^1(NH-CO-O-R^2)_n$$

formules dans lesquelles

$R^1$ représente un reste d'hydrocarbure aliphatique portant éventuellement des substituants inertes et/ou à non-saturation oléfinique et totalisant 2 à 18 atomes de carbone, un reste d'hydrocarbure cycloapliphatique portant éventuellement des substituants inertes et/ou à non-saturation oléfinique et totalisant 3 à 18 atomes de carbone, un reste d'hydrocarbure araliphatique portant éventuellement des substituants inertes et totalisant 7 à 18 atomes de carbone ou un reste d'hydrocarbure aromatique portant éventuellement des substituants inertes et totalisant 6 à 27 atomes de carbone,

$R^2$ représente un reste tel qu'on en obtient par élimination du groupe hydroxyle d'un alcool aliphatique, cycloaliphatique ou araliphatique primaire ou secondaire dont le point d'ébullition à la pression normale est inférieur ou supérieur d'au moins 10°C au point d'ébullition du polyisocyanate, des restes $R^2$ différents correspondant à cette définition pouvant se présenter dans la même molécule du carbamate à thermolyser et

n est égal à 2 ou à un nombre entier supérieur à 2,

éventuellement en présence de solvants inertes et, le cas échéant, en présence de catalyseurs, à des températures de 150 à 350°C et à des pressions de 0,001 à 20 bars et séparation subséquente des polyisocyanates et des alcools obtenus comme produits de décomposition, caractérisé en ce qu'on conduit la thermolyse en présence d'adjuvants choisis dans le groupe comprenant le chlorure d'hydrogène, des chlorures d'acides organiques, de substances à effet alkylant et des chlorures organostanniques.

2. Procédé suivant la revendication 1, caractérisé en ce qu'on utilise comme carbamates des carbamates de formule donnée dans le revendication 1 dans laquelle

$R^1$ représente un reste d'hydrocarbure aliphatique saturé ayant 6 à 10 atomes de carbone, un reste d'hydrocarbure cycloaliphatique saturé ayant 6 à 15 atomes de carbone, un reste xylyle ou un reste d'hydrocarbure aromatique totalisant 6 à 15 atomes de carbone, portant éventuellement des substituants méthyle,

$R^2$ est un reste d'hydrocarbure aliphatique saturé primaire ou secondaire ayant 1 à 4 atomes de carbone et

n est égal à 2.

3. Procédé suivant la revendication 1, caractérisé en ce qu'on utilise des carbamates de la formule mentionnée dans la revendication 1, pour lesquels

$R^1$ représente un reste tel qu'on l'obtient en éliminant les groupes isocyanato de l'hexaméthylènediisocyanate, du 1-isocanato-3,3,5-triméthyl-5-isocyanatométhylcyclohexane, du 2,4-diisocyanatotoluène, du 2,6-diisocyanatotoluène, du 2,4'-diisocyanatodiphénylméthane et/ou du 4,4'-diisocyanatodiphénylméthane et/ou de mélanges de ces deux isomères mentoinnés en dernier lieu avec leurs homologues supérieurs,

$R^2$ a la définition donnée dans la revendication 2.

4. Procédé suivant les revendications 1 à 3, caractérisé en ce qu'on utilise les adjuvants en une quantité de 0,001 à 5% en poids par rapport au mélange réactionnel total.